# EUROPEAN PATENT SPECIFICATION

(11) **EP 4 680 093 B1**
(45) Date of publication and mention of the grant of the patent: **02.12.2026**
(21) Application number: 23825630.9
(22) Date of filing: 13.12.2023
(51) Int. Cl.: A61B 5/00, A61N 5/06

(54) **METHOD AND SYSTEM FOR MONITORING MEDICAL TREATMENT OF TISSUE**
VERFAHREN UND SYSTEM ZUR ÜBERWACHUNG DER MEDIZINISCHEN BEHANDLUNG VON GEWEBE
PROCÉDÉ ET SYSTÈME DE SURVEILLANCE DE TRAITEMENT MÉDICAL D'UN TISSU

(30) Priority: 17.03.2023 US 202318185522
(43) Date of publication of application: 21.01.2026
(73) Proprietor: Modulight Corporation, 33101 Tampere (FI)
(72) Inventor: LEINONEN, Natalia, 33101 Tampere (FI); AUVINEN, Henri, 33101 Tampere (FI); LUOKKANEN, Lassi, 33101 Tampere (FI)
(74) Representative: Moosedog Oy
(86) International application number: PCT/FI2023/050691
(87) International publication number: WO 2024/194519

(56) References cited:
- WO-A1-2020/097186
- WO-A2-00/77491
- US-A1- 2011 270 056

## Description

### TECHNICAL FIELD

The present disclosure relates to methods for monitoring medical treatments of tissues. The present disclosure also relates to systems for monitoring medical treatments of tissues.

### BACKGROUND

With advancements in medicine and healthcare, medical treatments of malignant tissues such as cancerous tissues, tumours, neoplasm, and the like, are becoming more frequent than before. Such medical treatments include Photodynamic therapy (PDT), Photoimmunotherapy (PIT), and similar. Photodynamic therapy is a therapy involving activation of a photosensitizer by using a light of certain wavelength. PDT uses three components for effectively treating tumour in a tissue, the three components being: photosensitizer, light, and oxygen. Photosensitizers are molecules which absorb light of a certain wavelength and transfer energy from the light in the form of fluorescence, luminescence, and the like. This energy is utilized to kill target cells in the tissue (for example, such as tumour cells in the tissue).

Typically, biomedical laser devices are used to deliver laser light to tissue, for medical treatments. For the medical treatments to be successful, presence of a photosensitizer in the tissue is necessary. In existing techniques, a fluorescence spectrum is measured during treatment and in the fluorescence spectrum, presence of a peak indicating presence of fluorescence is checked manually. In other words, determining the presence of a photosensitizer depends on visual capability and expertise of a person viewing the fluorescence spectrum. It is required that the presence of the photosensitizer be detected throughout the medical treatments and that an amount of the photosensitizer reduce as the medical treatments progress. Non-reduction of the amount of the photosensitizer is indicative of other issues in the medical treatments (for example, such as lack of oxygen, insufficient irradiance, or similar, in PDT).

However, such existing methods and systems for determining whether a photosensitizer is present in tissue are unreliable and ineffective. Firstly, the detection of the presence of the photosensitizer just by seeing the fluorescence spectrum is difficult and depends highly on the visual capability and expertise of the person. Furthermore, a result of such detection varies from person to person. Moreover, such manual detection is a time-consuming process. Secondly, it is difficult to differentiate between a peak due to fluorescence emitted by the photosensitizer and a peak created by noise in the fluorescence spectrum. This difficulty in differentiation may be because of several reasons such as internal bleeding (since then blood absorbs more light that intended and the fluorescence spectrum becomes too noisy due to insufficient irradiance), spectrometer being too far from light emitting source, other physical conditions (such as temperature of tissue, oxygen saturation, and the like), and similar. Furthermore, very noisy spectrums require considerable pre-processing to be able to be interpreted by a human. False positives may result in ineffective medical treatments without the photosensitizer.

In a United States patent document US 2011/0270056 A1 (Linda R. Jones; "Determination of photodynamic therapy (pdt) treatment parameters"; assigned to College of Charleston Mayo Clinic in Florida), there are disclosed photodynamic therapy system and methods to determine the amount (concentration) of a photosensitizer agent present in the target tissue. The system also determines the tissue oxygenation. The system also determines light dosimetry parameters based on the amount of photosensitizer in the tissue and/or the tissue oxygenation.

Further, in a WIPO patent document WO 00/77491 A2 (Lothar Lilge; "Fiber optic multitasking probe"; assigned to Photonics Research Ontario), there is disclosed a multitasking optical fiber probe for collecting dosimeter information from more than one position in a sample. The basic principle of the present invention involves using one or more different sensor zones along the length of the fiber each with a different photoactive constituent having a sufficiently unique emission spectra (spectral or temporal) to enable deconvolution of the emission spectra by the computer and therefore correlation of the detected parameter with the position of the sensor zone along the length of the optical fiber. In the broadest form of the invention the probe is embodied by only one sensor zone located at some point along the length of the fiber spaced away from the end face of the fiber. More than one different photoactive constituent could be incorporated into a single sensor zone for measuring several factors in the vicinity of the sensor zone.

Further, in a WIPO patent document WO 2020/097186 A1 (Timothy C. ZHU; "Methods and systems for photodynamic therapy calculations"; assigned to University of Pennsylvania Penn), there are disclosed methods and systems for implementing a photodynamic therapy. An example method comprises determining, during photodynamic therapy and using a plurality of optical probes spatially distributed within a patient, data indicative of one or more of a photodynamic therapy dosage, a fluence rate of a photodynamic therapy treatment light, or a reactive oxygen species concentration associated with corresponding locations of the plurality of optical probes. The example method also comprises changing, based on the data, one or more treatment parameters associated with providing a photodynamic therapy.

Therefore, in the light of the foregoing discussion, there exists a need to overcome the aforementioned drawbacks associated with existing techniques for monitoring the medical treatment of the tissue (and in particular, presence of the photosensitiser in the tissue).

### SUMMARY

The present disclosure seeks to provide a method for monitoring a medical treatment of a tissue. The present disclosure also seeks to provide a system for monitoring a medical treatment of a tissue. An aim of the present disclosure is to provide a solution that overcomes at least partially the problems encountered in prior art.

In one aspect, an embodiment of the present disclosure provides a method for monitoring a medical treatment of a tissue, the method comprising:
- receiving, by at least one processor, a fluorescence spectrum that is measured while the tissue is illuminated by laser light emitted by a biomedical laser device, wherein the fluorescence spectrum corresponds to a variation of an intensity of light emitted by the photosensitizer when the tissue is illuminated by laser light emitted by the biomedical laser device;
- obtaining, by the at least one processor, reference fluorescence data from a digital library of fluorescence data, based at least on a type of a photosensitizer that is required to be present in the tissue for the medical treatment;
- determining, by the at least one processor, a region of interest in the measured fluorescence spectrum, based on a reference fluorescence spectrum in the reference fluorescence data;
- determining, by the at least one processor, whether a signal to noise ratio of the measured fluorescence spectrum in the region of interest is greater than a first threshold;
- when it is determined that the signal to noise ratio of the measured fluorescence spectrum in the region of interest is greater than the first threshold, determining, by the at least one processor, a correlation coefficient that is indicative of a correlation between the region of interest in the measured fluorescence spectrum and the reference fluorescence spectrum;
- determining, by the at least one processor, whether the correlation coefficient is greater than a second threshold; and
- when it is determined that the correlation coefficient is greater than the second threshold, determining, by the at least one processor that the photosensitizer is present in the tissue.

In another aspect, an embodiment of the present disclosure provides a system for monitoring a medical treatment of a tissue, the system comprising at least one processor configured to:
- receive a fluorescence spectrum that is measured while the tissue is illuminated by laser light emitted by a biomedical laser device, wherein the fluorescence spectrum corresponds to a variation of an intensity of light emitted by the photosensitizer when the tissue is illuminated by laser light emitted by the biomedical laser device;
- obtain reference fluorescence data from a digital library of fluorescence data, based at least on a type of a photosensitizer that is required to be present in the tissue for the medical treatment;
- determine a region of interest in the measured fluorescence spectrum, based on a reference fluorescence spectrum in the reference fluorescence data;
- determine whether a signal to noise ratio of the measured fluorescence spectrum in the region of interest is greater than a first threshold;
- when it is determined that the signal to noise ratio of the measured fluorescence spectrum in the region of interest is greater than the first threshold, determine a correlation coefficient that is indicative of a correlation between the region of interest in the measured fluorescence spectrum and the reference fluorescence spectrum;
- determine whether the correlation coefficient is greater than a second threshold; and
- when it is determined that the correlation coefficient is greater than the second threshold, determine that the photosensitizer is present in the tissue.

Embodiments of the present disclosure substantially eliminate or at least partially address the aforementioned problems in the prior art and enable effective and efficient monitoring of the medical treatment of the tissue to accurately and reliably determine whether or not a photosensitizer (that is required for the medical treatment) is present in the tissue.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:
FIG. 1 illustrates a block diagram of an architecture of a system for monitoring a medical treatment of a tissue, in accordance with an embodiment of the present disclosure;
FIG. 2 illustrates an exemplary environment in which a system for monitoring a medical treatment of a tissue is used, in accordance with an embodiment of the present disclosure;
FIG. 3 illustrates an architecture of a biomedical laser device, in accordance with an embodiment of the present disclosure;
FIGs. 4A and 4B collectively illustrate a method for monitoring a medical treatment of a tissue, in accordance with an embodiment of the present disclosure;
FIG. 5 illustrates exemplary spectrums corresponding to a pre-treatment phase of a tissue and to a post-treatment phase of the tissue, in accordance with an embodiment of the present disclosure;
FIGs. 6A, 6B, and 6C illustrate a first exemplary set of graphs for determining whether a photosensitizer is present in a tissue, in accordance with an embodiment of the present disclosure;
FIGs. 7A, 7B, and 7C illustrate a second exemplary set of graphs for determining whether a photosensitizer is present in a tissue, in accordance with an embodiment of the present disclosure;
FIGs. 8A, 8B, and 8C illustrate a third exemplary set of graphs for determining whether a photosensitizer is present in a tissue, in accordance with an embodiment of the present disclosure; and
FIGs. 9A, 9B, and 9C illustrate a fourth exemplary set of graphs for determining whether the photosensitizer is present in a tissue, in accordance with an embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In one aspect, an embodiment of the present disclosure provides a method for monitoring a medical treatment of a tissue, the method comprising:
- receiving, by at least one processor, a fluorescence spectrum that is measured while the tissue is illuminated by laser light emitted by a biomedical laser device, wherein the fluorescence spectrum corresponds to a variation of an intensity of light emitted by the photosensitizer when the tissue is illuminated by laser light emitted by the biomedical laser device;
- obtaining, by the at least one processor, reference fluorescence data from a digital library of fluorescence data, based at least on a type of a photosensitizer that is required to be present in the tissue for the medical treatment;
- determining, by the at least one processor, a region of interest in the measured fluorescence spectrum, based on a reference fluorescence spectrum in the reference fluorescence data;
- determining, by the at least one processor, whether a signal to noise ratio of the measured fluorescence spectrum in the region of interest is greater than a first threshold;
- when it is determined that the signal to noise ratio of the measured fluorescence spectrum in the region of interest is greater than the first threshold, determining, by the at least one processor, a correlation coefficient that is indicative of a correlation between the region of interest in the measured fluorescence spectrum and the reference fluorescence spectrum;
- determining, by the at least one processor, whether the correlation coefficient is greater than a second threshold; and
- when it is determined that the correlation coefficient is greater than the second threshold, determining, by the at least one processor, that the photosensitizer is present in the tissue.

In another aspect, an embodiment of the present disclosure provides a system for monitoring a medical treatment of a tissue, the system comprising at least one processor configured to:
- receive a fluorescence spectrum that is measured while the tissue is illuminated by laser light emitted by a biomedical laser device, wherein the fluorescence spectrum corresponds to a variation of an intensity of light emitted by the photosensitizer when the tissue is illuminated by laser light emitted by the biomedical laser device;
- obtain reference fluorescence data from a digital library of fluorescence data, based at least on a type of a photosensitizer that is required to be present in the tissue for the medical treatment;
- determine a region of interest in the measured fluorescence spectrum, based on a reference fluorescence spectrum in the reference fluorescence data;
- determine whether a signal to noise ratio of the measured fluorescence spectrum in the region of interest is greater than a first threshold;
- when it is determined that the signal to noise ratio of the measured fluorescence spectrum in the region of interest is greater than the first threshold, determine a correlation coefficient that is indicative of a correlation between the region of interest in the measured fluorescence spectrum and the reference fluorescence spectrum;
- determine whether the correlation coefficient is greater than a second threshold; and
- when it is determined that the correlation coefficient is greater than the second threshold, determine that the photosensitizer is present in the tissue.

The present disclosure provides the aforementioned method and the aforementioned system for monitoring the medical treatment of the tissue. The aforementioned method incorporates mathematical computations, so that the determination of presence or absence of the photosensitizer becomes easy, fast, accurate and reliable. The aforementioned method and the aforementioned system eliminate manual detection of the photosensitizer, hence reducing a time taken to determine whether the photosensitizer is present, increasing accuracy of such a determination, increasing repeatability of the determination, and similar benefits. The aforementioned method employs computation of the correlation coefficient and comparison of the correlation coefficient with respect to the second threshold, for enabling accurate and reliable differentiation between a noise peak and a peak due to fluorescence emitted by the photosensitizer, in the measured fluorescence spectrum.

Optionally, the medical treatment is at least one of: Photodynamic therapy (PDT), Photoimmunotherapy (PIT), Photothermal therapy (PTT), Photochemotherapy. Optionally, in this regard, the tissue is a body tissue of a human and/or an animal. For example, the tissue may be a brain tissue, a kidney tissue, a lung tissue, a skin tissue, and the like. It will be appreciated that monitoring of such medical treatments is required for ensuring patient safety and treatment efficacy.

Throughout the present disclosure, the term *"biomedical laser device"* refers to a device which uses the laser light to provide the medical treatment to the tissue. The biomedical laser device can also be employed for purpose of other biomedical applications (for example, such as biomedical diagnosis). Optionally, the biomedical laser device comprises a laser light emitter that, in operation, emits the laser light; at least one light guide for transmitting the laser light from the laser light emitter to the tissue; and a coupling arrangement for optically coupling the laser light emitter with the at least one light guide. Optionally, the laser light emitter comprises at least one light-emitting element that is controllable to emit the laser light of at least one target wavelength. The at least one light-emitting element may, for example, be a semiconductor laser. Optionally, the coupling arrangement is at least one element that provides a leak proof connection between the laser light emitter and the at least one light guide. This connection may be a detachable connection. Optionally, the at least one light guide is implemented as at least one of: an optical fibre, an optical waveguide. As an example, the at least one light guide may comprise 2-8 light guides. Optionally, a given light guide is capable of emitting the laser light and is also capable of measuring the fluorescence spectrum. This is possible when appropriate filters are applied (for example, in the at least one light guide) to prevent saturation in a wavelength of the laser light that is emitted. Optionally, when the at least one light guide comprises a plurality of light guides, at a given time, at least one of the plurality of light guides directs the laser light towards the tissue while at least one remaining light guide of the plurality of light guides acts as a spectrometer for measuring the fluorescence spectrum.

Optionally, the system further comprises a spectrometer for measuring the fluorescence spectrum.

The term *"fluorescence spectrum"* refers to a graphical representation of an intensity of light emitted by a substance after it has been excited by absorbing light of a specific wavelength. In this case, the light which is absorbed is the laser light, the specific wavelength of the laser light is selected according to specifications of the medical treatment and patient's medical condition. Furthermore, the substance is the photosensitizer, and thus the light emitted by the photosensitizer is fluorescence. Notably, the at least one light guide of the biomedical laser device is arranged in proximity of the tissue subjected to the medical treatment. Moreover, the at least one light guide or the at least one of the plurality of light guides, directs the laser light of a specific wavelength towards the tissue, this laser light is absorbed by the photosensitizer (if present), the photosensitizer after absorbing the emitted light attains an excited state and returns back to its original state, and in the process of returning back to its original state, the photosensitizer emits light. Moreover, the at least one light guide or the at least one remaining light guide acts as a receiver/sensor to receive light (i.e., fluorescence) emitted by the photosensitizer. Furthermore, a variation of an intensity of the light emitted by the photosensitizer with respect to wavelength of the light emitted by the photosensitizer, is understood to be the fluorescence spectrum (that is measured).

Optionally, the method further comprises processing the measured fluorescence spectrum, by at least one of:
- removing dark spectrum from the measured fluorescence spectrum,
- removing ambient light spectrum from the measured fluorescence spectrum,
- low pass filtering of the measured fluorescence spectrum.

In this regard, the step of processing of the measured fluorescence spectrum is a pre-processing step that is implemented prior to subsequent processing of the measured fluorescence spectrum (for detecting whether the photosensitizer is present in the tissue). Such processing of the measured fluorescence spectrum improves a quality of the measured fluorescence spectrum, which further leads to an improvement in an accuracy of detection of presence of the photosensitizer in the tissue.

The term *"dark spectrum"* refers to that portion of a measured spectrum of the laser light which is present when no light is received by the at least one light guide or the at least one remaining light guide (which serves as the spectrometer) during measurement of the fluorescence spectrum. Thus, the dark spectrum is essentially a background noise level of all measurements with a same spectrometer integration time. The dark spectrum is an unwanted spectrum, which if present along with the measured fluorescence spectrum, would result in errors when processing and interpreting the measured fluorescence spectrum subsequently. Hence, removing the dark spectrum improves the quality of the measured fluorescence spectrum, and improves an accuracy of the method.

The term *"ambient light spectrum"* refers to a spectrum of light which is present in an environment where the biomedical laser device is in use and/or a spectrum of light guide signals when the biomedical laser device is inactive (or switched off). This light which is present in the environment of using the biomedical laser device could emanate from a natural light source (for example, such as the sun) and/or an artificial light source (for example, such as a tube light). The ambient light spectrum may be measured to determine a broader base level with additional noise introduced by the environment of measurement, and not just noise introduced by the spectrometer itself. The ambient light spectrum is an unwanted spectrum which can have an adverse impact on efficacy of the medical treatment, and the ambient light spectrum can interfere with the photosensitizer to produce unwanted reactions and to produce incorrect measurement data while the fluorescence spectrum is measured. Hence, removing the ambient light spectrum improves the quality of the measured fluorescence spectrum, and improves the accuracy of the method.

The low pass filtering of the measured fluorescence spectrum is optionally performed, for removing noise from the measured fluorescence spectrum. The noise could be at least one of noise from the biomedical laser device, noise from the natural light source and/or from the artificial light source, noise due to internal bleeding of the region where medical treatment is being performed, noise from at least one sensor arranged in proximity of the tissue, and similar. As an example, the noise from the biomedical laser device may include noise due to incorrect placement of the at least one light guide in proximity of the tissue. For example, one exemplary scenario of such incorrect placement may be when the plurality of light guides are used, such that the at least one of the plurality of light guides which directs the laser light towards the tissue is too far away from the at least one remaining light guide which acts as the spectrometer. Either the dark spectrum or the ambient light spectrum can be used to determine noise level, but both may not be used at the same time. It would become difficult to process the measured fluorescence spectrum due to the presence of the noise and the noise would also make interpretation of results difficult and error-prone. Hence, the removal of the noise from the measured florescence spectrum is important for accurately determining whether the photosensitizer is present in the tissue.

The term *"reference fluorescence data"* refers to a portion of fluorescence data (stored in the digital library) that is used as a frame of reference for monitoring the medical treatment of the tissue. Optionally, the fluorescence data stored in the digital library comprises historical measured fluorescence spectrums. Herein, the reference fluorescence data is pre-stored in the digital library, which is a digital database of reference fluorescence data for various medical treatments of various tissues.

Throughout the present disclosure, the term *"photosensitizer"* refers to a biochemical substance which is sensitive towards a given range of wavelengths of light, and when light having a wavelength belonging to the given range of wavelengths is incident upon the photosensitizer, the photosensitizer absorbs the light and responds by producing a biochemical reaction. It will be appreciated that photosensitizers have various types depending on their chemical composition and their sensitivity for various ranges of wavelengths. Furthermore, different types of photosensitizers would produce different measured fluorescence spectrums. Optionally, the digital library of fluorescence data comprises historical measured fluorescence spectrums for different types of photosensitizers. Some exemplary types of photosensitizers may be hematoporphyrin derivatives, phenothiazine, cyanine, phytotherapeutic agents, phthalocyanine, and the like. For example, three types of photosensitizers A, B and C may be suitable for performing a medical treatment of a brain tissue. In such a case, the photosensitizer of type A may presently be used for performing the medical treatment of the brain tissue. Therefore, the digital library is accessed for obtaining historical measured fluorescence spectrum(s) associated with the medical treatment of the brain tissue using the photosensitizer of type A.

Optionally, the step of obtaining the reference fluorescence data from the digital library of fluorescence data is based also on a type of the tissue. Furthermore, the type of tissue comprises any one of: connective tissue, epithelial tissue, muscle tissue, and nervous tissue. The type of the tissue could be pre-known for a patient, be provided as an input by an operator of the biomedical laser device, or similar. The type of the tissue would affect the fluorescence spectrum produced by the photosensitizer, and thus it is beneficial to take the type of the tissue into account when obtaining the reference fluorescence data.

Optionally, the method further comprises receiving sensor data that is measured by at least one sensor arranged in proximity of the tissue, the sensor data comprising at least one of: a temperature of the tissue, an oxygen saturation of the tissue, a distance between light guides in proximity of the tissue, wherein the step of obtaining the reference fluorescence data from the digital library of fluorescence data is based also on the sensor data.

In this regard, the sensor data is measured to determine physical conditions corresponding to the medical treatment of the tissue. Furthermore, the at least one sensor is arranged in proximity of the tissue to measure the sensor data accurately. The at least one sensor is described later in more detail. It will be appreciated that the measured fluorescence spectrum would depend upon each of the above-mentioned physical conditions, such that even when one of such conditions changes while the rest are constant, the fluorescence spectrum changes considerably. Therefore, in order to obtain most-relevant reference fluorescence data from the digital library, the sensor data indicative of the above-mentioned conditions is taken into account. Herein, greater a similarity between present physical conditions and physical conditions associated with the reference fluorescence data, greater will be efficacy of the method. All the above-mentioned physical quantities act as important criterions for obtaining the reference fluorescence data from the digital library. For example, the digital library may comprise five historical measured fluorescence spectrums for a given photosensitizer used in photodynamic therapy of a given tissue, corresponding to five different temperatures of the given tissue. In such a case, a historical measured fluorescence spectrum corresponding to a temperature that is closest to a present temperature of the given tissue, is obtained as the reference fluorescence data from the digital library.

Optionally, the method further comprises creating the digital library of fluorescence data using at least historical measured fluorescence spectrums, wherein the historical measured fluorescence spectrums are measured historically by administering different types of photosensitizers in the tissue. It will be appreciated that for the medical treatment of the tissue to be successful, it is important to have the reference fluorescence data, as the reference fluorescence data is used for the determination of presence or absence of the photosensitizer. Therefore, the creation of the digital library of the fluorescence data is done prior to performing processing steps for determining presence or absence of the photosensitizer. The digital library comprises the historical measured fluorescence spectrums for different types of photosensitizers in the tissue. The florescence data comprises at least these historical measured fluorescence spectrums, which are measured when it is known that their corresponding photosensitizer is present for sure in the tissue. For obtaining each historical measured fluorescence spectrum, a medical practitioner and/or the operator of the biomedical laser device administers a given type of photosensitizer in the tissue and uses the biomedical laser device to measure a corresponding fluorescence spectrum. The digital library is stored on a data repository, wherein the data repository can be a local storage device communicably coupled to the biomedical laser device, a removable storage device, a cloud-based storage, or similar. Optionally, the digital library comprises at least the reference fluorescence data. The reference fluorescence data may include one or more of the historical measured fluorescence spectrums. It will be appreciated that a single digital library may serve multiple biomedical laser devices, and in such a case, the data repository can, for example, be the cloud-based storage.

Optionally, the historical measured fluorescence spectrums are measured historically for at least one of: different temperatures of the tissue, different oxygen saturations in the tissue, different distances between light guides in proximity of the tissue. As mentioned previously, fluorescence spectrums produced by photosensitizers depend on each of these physical conditions, so change of any of these physical conditions impacts the fluorescence spectrums considerably. Thus, recordal and storage of the historical measured fluorescence spectrums corresponding to variations in at least one of these physical conditions, enables in creation of a data-rich digital library which includes detailed and relevant fluorescence data for various real-world use situations.

It will be appreciated that for a given photosensitizer that is administered in the tissue, when a given physical condition changes, its corresponding fluorescence spectrum changes. In this regard, the different values/states of the given physical condition may be employed when creating the digital library, so that in addition to including how the different types of photosensitizers affect the measured fluorescence spectrums, the digital library also includes how variation of the given physical condition affects the measured fluorescence spectrums. In this way, the digital library becomes more enriched, and highly relevant reference fluorescence data can be obtained from it. In this regard, the given physical condition is at least one of: the temperature of the tissue, the oxygen saturation of the tissue, the distance between light guides in proximity of the tissue.

Optionally, over time, the digital library is populated with a plurality of peak shapes for fluorescence emitted by a plurality of photosensitizers at different temperatures of the tissue, at different tissue oxygen saturations, at different type of tissue, and the different distances between the light guides in the proximity of the tissue. The plurality of peak shapes may be stored in the form of images, in the digital library.

Optionally, the method further comprises extracting the region of interest in the measured fluorescence spectrum, from an entirety of the measured fluorescence spectrum. The term *"extracting"* here refers to cropping out the region of interest from an entirety of the measured fluorescence spectrum. Beneficially, extracting the region of interest in the measured fluorescence spectrum simplifies the processing required in the method.

Throughout the present disclosure, the term *"region of interest"* refers to that part of the measured fluorescence spectrum which represents fluorescence emitted by the photosensitizer. In this regard, the fluorescence emitted by the photosensitizer may be shaped as a peak in the measured fluorescence spectrum. Higher the peak, greater is the fluorescence emitted by the photosensitizer. Therefore, the region of interest may be a region representing the peak of fluorescence (i.e., fluorescence peak) in the measured fluorescence spectrum. The region of interest corresponds to a range of wavelengths at which the photosensitizer emits fluorescence. By determining the region of interest, the determination of presence or absence of photosensitizers becomes simple and accurate, as the region of interest can be further processed easily to ascertain whether it truly represents presence of the photosensitizer in the tissue or not. The region of interests for the different types of photosensitizers are different, as the measured fluorescence spectrums for the different types of photosensitisers are also different.

Optionally, extracting the region of interest in the measured fluorescence spectrum makes the storage of the measured fluorescence spectrum (when the photosensitizer determined to be present) in the digital library memory-efficient. The measured fluorescence spectrums stored in the digital library can be used as the reference fluorescence spectrums for the medical treatments of the tissue in future.

It will be appreciated that the first threshold is a parameter with which the signal to noise ratio of the measured fluorescence spectrum in the region of interest is compared, so as to accurately determine whether there is enough signal strength in the measured fluorescence spectrum (in the region of interest) to confidently know that noise does not create false positives. In other words, by such comparison, it is determined whether enough signal intensity of fluorescence has been emitted to indicate that the photosensitizer is likely present in the tissue. When the signal to noise ratio of the measured fluorescence spectrum in the region of interest is greater than the first threshold, it means that the signal strength of the fluorescence (i.e., the intensity of the fluorescence that has been emitted) is sufficient to indicate that the photosensitizer is likely present in the tissue.

Optionally, the method further comprises determining the first threshold by:
- determining root mean square (RMS) values for a noise level and a signal level of the region of interest in the measured fluorescence spectrum;
- determining the signal to noise ratio of the measured fluorescence spectrum in the region of interest, based on the RMS values for the noise level and the signal level; and
- dynamically setting the first threshold, based on the signal to noise ratio.

Optionally, in this regard, the first threshold is equal to the signal to noise ratio. The RMS values for the noise level and the signal level, and the signal to noise ratio of the measured fluorescence spectrum may be determined using well-known approaches for the same. The RMS noise level may, for example, be calculated from the dark spectrum or the ambient spectrum measurement. As an example, the RMS value for the noise level may lie in a range of 200-1500 spectrometer measurement units (for example, counts/microwatts) and the RMS value for the signal level may lie in a range of 225-16000 units. These levels may produce signal to noise ratios lying in a range of 1.25 to 6400. For example, the first threshold may be set at 1.5, so that only that measured fluorescence spectrum which has SNR greater than 1.5 in the region of interest, will be accepted for further processing. It will be appreciated that the first threshold could change based on further analysis of measured fluorescence spectrums, other indications, and similar. Optionally, the first threshold is provided as an input from the digital library. Alternatively, optionally, the first threshold is pre-determined and coded into processing software.

Optionally, the method further comprises normalizing the measured fluorescence spectrum according to the reference fluorescence spectrum, prior to the step of determining the correlation coefficient. The term *"normalizing"* used herein refers to a processing operation in which a scale of a given spectrum is adjusted to correspond to a scale of another spectrum. Normalization enables in accurately determining the correlation between the region of interest of the measured fluorescence spectrum and the reference fluorescence spectrum. In particular, the normalization enables intensities for the measured fluorescence spectrum and the reference fluorescence spectrum, corresponding to a similar range of wavelengths, to become comparable. Normalization is performed using well-known data processing techniques. Alternatively, optionally, the method further comprises normalizing the reference fluorescence spectrum according to the measured fluorescence spectrum, prior to the step of determining the correlation coefficient.

The correlation coefficient is indicative of the correlation (i.e., an interrelationship) between the region of interest of the measured fluorescence spectrum and the region of interest of the reference fluorescence spectrum, such that a high value of the correlation coefficient indicates a high degree of such correlation. Optionally, the correlation coefficient is a Pearson correlation coefficient. The Pearson correlation coefficient typically has a value ranging between -1 to 1. Optionally, the correlation coefficient has a value lying in a range of 0 to 1. Other ranges of the correlation coefficient are also feasible. Optionally, the correlation coefficient is determined using at least one of: a mathematical formula, an algorithm, a graphical processing operation. Other ways of determining the correlation are well within the scope of the present disclosure. It will be appreciated that the value of the correlation coefficient indicates how closely related the region of interest is with the reference fluorescence spectrum, since a close relationship therebetween is indicative of the presence of the photosensitizer in the tissue. For example, the value of the correlation coefficient indicates an extent of matching between the peak representing fluorescence emission in the region of interest and a corresponding peak in the reference fluorescence spectrum. Such matching may be in terms of peak shape, peak size, and similar.

The correlation coefficient is compared with the second threshold, in order to determine whether there exists enough correlation between the region of interest and the reference fluorescence spectrum to deduce that the photosensitizer is present in the tissue. The second threshold can be obtained from the reference fluorescence data (in which case, the second threshold is stored in the digital library), be entered as an input entered by the operator operating the biomedical laser device, or the like. Optionally the second threshold lies in the range of 0.75 to 0.95. As an example, the second threshold may be from 0.75, 0.76, 0.78, 0.82 or 0.87 up to 0.80, 0.85, 0.90, 0.925 or 0.95.

It will be appreciated that when the correlation coefficient is greater than the second threshold, it means that there is a high degree of correlation between the region of interest and the reference fluorescence spectrum.

This means, for example, that the peak representing the fluorescence emission in the region of interest closely matched the corresponding peak in the reference fluorescence spectrum. In such a case, it is determined that the photosensitizer is present in the tissue. In other words, higher the value of the correlation coefficient, greater is a likelihood of the photosensitizer being present in the tissue. An accuracy of such determination is high, since the method evaluates both the signal to noise ratio of the measured fluorescence spectrum and the correlation coefficient with respect to their corresponding thresholds, and only when both the signal to noise ratio and the correlation coefficient are greater than their corresponding thresholds, does the method deduce that the photosensitizer is present in the tissue. By such an approach, false positives are minimised.

Optionally, when it is determined that the photosensitizer is present in the tissue, the method further comprises providing an indication of the photosensitizer being present in the tissue, the indication being at least one of: a visual indication, an audio indication, a haptic indication. In this regard, provision of the indication enables the operator of the biomedical laser device to know that the photosensitizer is present in the tissue, so that the operator can continue the medical treatment. The indication can be provided in one or more of various feasible forms, such as visually, auditory, haptic signals, and the like. Optionally, the indication of the photosensitizer being present in the tissue is provided on at least one of: the biomedical laser device, a device associated with an operator of the biomedical laser device. In this regard, at least one of: the biomedical laser device, the device associated with the operator of the biomedical laser device, optionally comprises at least one indicator, wherein the indication is provided by controlling the at least one indicator. Optionally, the at least one indicator comprises at least one of: a display, a light-emitting element (such as a light-emitting diode (LED)), a buzzer, a speaker, a haptic feedback device. Examples of the device associated with the operator of the biomedical laser device include, but are not limited to, a smartphone, a smart watch, a smart band, a laptop computer, a tablet computer, and a personal digital assistant.

In an example, the indication of the photosensitizer being present in the tissue may be blinking of an LED in a certain manner, emission of a certain colour of light, and similar. In another example, the indication of the photosensitizer being present in the tissue may be a specific sound from the buzzer. In yet another example, the indication of the photosensitizer being present in the tissue may be displaying of at least one image on the display. Moreover, displaying of at least one image also covers displaying of a video, as the video is a sequence of images. In still another example, the indication of the photosensitizer being present in the tissue may be displaying a text on the display, wherein the text can be a specific code, a message, or the like. In yet another example, the indication of the photosensitizer being present in the tissue may be vibration of a haptic feedback device connected to the medical laser.

Optionally, when it is determined that the photosensitizer is present in the tissue, the method further comprises sending measured fluorescence data to a data repository at which the digital library of fluorescence data is maintained, for storing the measured fluorescence data in the digital library, wherein the measured fluorescence data comprises the measured fluorescence spectrum, the type of the photosensitizer present in the tissue, and the correlation coefficient that is determined. In such a case, the presence of the photosensitizer in the tissue is confirmed using the method, and thus the measured fluorescence data can beneficially serve as a reference fluorescence data for a future use of the method. When, at a future instance of use of the method, the type of photosensitizer is same as that in the measured fluorescence data, the measured fluorescence data may be obtained as reference fluorescence data for determining presence of that photosensitizer in the tissue. Thus, the measured fluorescence data is beneficially sent to the data repository, for enriching the digital library with potentially useful fluorescence data. The measured fluorescence data may be sent over a communication network between the at least one processor implementing the method and the data repository. Optionally, when it is determined that the photosensitizer is present in the tissue, the method further comprises including the sensor data in the measured fluorescence data.

Optionally, when it is determined that the signal to noise ratio of the measured fluorescence spectrum in the region of interest is lesser than or equal to the first threshold, the method further comprises determining that the photosensitizer is absent in the tissue. The signal to noise ratio of the measured fluorescence spectrum in the region of interest being lesser than or equal to the first threshold means that an amount of the fluorescence measured in the measured fluorescence spectrum is insufficient to indicate the presence of the photosensitizer. This means, for example, that the peak representing fluorescence emission in the region of interest is too weak to indicate the presence of the photosensitizer in the tissue. Therefore, in such a case, the absence of the photosensitizer is determined accurately, using the method.

Optionally, when it is determined that the correlation coefficient is lesser than or equal to the second threshold, the method further comprises determining that the photosensitizer is absent in the tissue. It will be appreciated that when the correlation coefficient is lesser than or equal to the second threshold, it means that a degree of correlation between the region of interest in the measured fluorescence spectrum and the reference fluorescence spectrum, is low. This means, for example, that the peak representing the fluorescence emission in the region of interest mismatches with the corresponding peak in the reference fluorescence spectrum. In such a case, it is accurately determined that the photosensitizer is absent in the tissue, using the method.

Optionally, when it is determined that the photosensitizer is absent in the tissue, the method further comprises providing an indication of the photosensitizer being absent in the tissue. In this case, the indication is at least one of: a visual indication, an audio indication, a haptic indication, wherein the indication of the photosensitizer being absent in the tissue is different from the indication of the photosensitizer being present in the tissue. Optionally, the indication of the photosensitizer being absent in the tissue is provided by controlling the at least one indicator. For example, the visual indication of the photosensitizer being absent in the tissue may be that an LED emits red light, whereas the visual indication of the photosensitizer being present in the tissue may be that the LED emits green light.

Optionally, the indication of the photosensitizer being absent in the tissue is provided by the at least one indicator which is provided on at least one of: the biomedical laser device, a device associated with an operator of the biomedical laser device.

Optionally, when it is determined that the photosensitizer is absent in the tissue, the method further comprises at least one of:
- prompting an operator of the biomedical laser device to switch off or disable the biomedical laser device and/or to administer the photosensitizer in the tissue;
- generating a control signal for switching off or disabling the biomedical laser device;
- generating a control signal for a machine to administer the photosensitizer in the tissue.

In this regard, at least one of the aforementioned steps enable in implementing control measures when the photosensitizer is detected to be absent. These control measures are important for ensuring patient safety and/or treatment efficacy. When the biomedical laser device is switched off or disabled, the medical treatment is paused or stopped, thereby preventing unnecessary exposure of the patient to radiation in the absence of the photosensitizer. Such switching off or disabling of the biomedical laser device can be performed by the operator (upon receiving the prompt) and/or automatically by the at least one processor implementing the method. When a control measure of administering the photosensitizer in the tissue is implemented, then the medical treatment may be continued (for example, after waiting for a given time period in which the photosensitizer reaches the tissue). Optionally, the administering of the photosensitizer in the tissue is done by injecting the photosensitizer of a particular type and of a particular amount in the tissue of the patient. Alternatively, optionally, the administering of the photosensitizer in the tissue is done by administering an oral dosage of the photosensitizer of a particular type and of a pre-known amount to the patient.

Optionally, the process of administering of the photosensitizer is performed by the operator. Alternatively, optionally, the process of administering of the photosensitizer is performed by the machine. Examples of the machine could be a robot, a robotic arm, and the like. Furthermore, the machine is operated by a control signal. The control signal can be at least one of: a voltage signal, a current signal.

Optionally, at least one machine learning algorithm is employed to analyse the measured fluorescence spectrum. Optionally, in this regard, the analysis of the measured fluorescence spectrum is employed to iteratively suggest, using the at least one machine learning algorithm, at least one of: a value of the first threshold, a feature from the measured fluorescence spectrum. Optionally, the at least one machine learning algorithm enables correlation of the analysis of the measured fluorescence spectrum with an efficacy of the medical treatment. The analysis of the measured fluorescence spectrum could also be used for generating and/or improving visualizations of the measured fluorescence spectrums.

Optionally, the method further comprises processing at least one of: the measured fluorescence spectrum, additional spectral data, to determine at least one of: the temperature of the tissue, the oxygen saturation of the tissue, the distance between light guides in proximity of the tissue. In this way, at least one of: the measured fluorescence spectrum, the additional spectral data, can beneficially be employed for determining various physical conditions related to the tissue, and thus this enables in a comprehensive monitoring of the medical treatment of the tissue. Such monitoring facilitates higher treatment efficacy, patient safety, and similar.

As an example, the additional spectral data for determining the temperature of the tissue may be captured by illuminating the tissue with the laser light and measuring that portion of the laser light which is reflected by the tissue. Wavelength(s) corresponding to the portion of the laser light which is reflected by the tissue may be indicative of the temperature of the tissue. Upon processing such additional spectral data, the temperature of the tissue can be determined accurately and easily. As another example, the additional spectral data for determining the oxygen saturation of the tissue may be captured by illuminating the tissue with two different wavelengths of laser light, and by measuring light in certain spectral ranges. The analysis of such measurement (i.e., the additional spectral data) enables in determination of the oxygen saturation of the tissue. As yet another example, the additional spectral data for determining the distance between light guides in proximity of the tissue (when the biomedical laser device comprises the plurality of light guides) may be captured by measuring intensity variation in tissue absorption (of the emitted laser light) at a certain wavelength or a certain range of wavelengths. The analysis of such measurement (i.e., the additional spectral data) enables in determination of the distance between light guides in proximity of the tissue.

The present disclosure also relates to the system. Various embodiments and variants disclosed above, with respect to the aforementioned method, apply *mutatis mutandis* to the system.

The at least one processor implements the method described above. The at least one processor is implemented as hardware, software, firmware, or a combination of these. In some implementations, the at least one processor refers to a single processor, whereas in other implementations, the at least one processor refers to a plurality of processors.

Optionally, the system further comprises a data repository communicably coupled to the at least one processor, wherein the digital library of fluorescence data is maintained at the data repository.

In this regard, the data repository is communicably coupled to at least one processor via a communication network. Examples of the communication network include, but are not limited to one or more of a wireless network (for example, such as a wireless fidelity (WiFi) network, a Bluetooth network, a radio network, a cellular network, and the like), and a wired network (for example, such as an Ethernet network, and similar).

Optionally, in the system, the at least one processor is communicably coupled to the biomedical laser device and/or a device associated with an operator of the biomedical laser device, and wherein when it is determined that the photosensitizer is present in the tissue, the at least one processor is further configured to provide an indication of the photosensitizer being present in the tissue on the biomedical laser device and/or the device associated with an operator of the biomedical laser device, the indication being at least one of: a visual indication, an audio indication, a haptic indication. In this regard, the at least one processor is communicably coupled to the biomedical laser device and/or a device associated with the operator via a communication network. Examples of such a communication network are mentioned above.

Optionally, the system further comprises the at least one indicator, wherein the at least one indicator is provided on at least one of: the biomedical laser device, the device associated with the operator of the biomedical laser device. The at least one indicator can be at least one of: a visual indicator, an audio indicator, a haptic indicator. In an example, a visual indicator comprises at least one of: a display, a light-emitting element. In another example, an audio indicator comprises at least one of: a buzzer, a speaker. In yet another example, a haptic indicator comprises a haptic feedback device.

Optionally, in the system, the biomedical laser device comprises the at least one processor. In this regard, the system is implemented in the biomedical laser device. It will be appreciated that such an implementation of the system is space-efficient, cost-efficient, and easy to use. Therefore, in such a case, the biomedical laser has the capability to monitor the medical treatment of the tissue accurately.

Optionally, the system further comprises at least one sensor communicably coupled to the at least one processor, the at least one sensor comprising at least one of: a temperature sensor, an oxygen saturation sensor, a distance sensor, the at least one sensor being arranged in proximity of the tissue, and wherein the at least one processor is further configured to receive sensor data that is measured by the at least one sensor, the sensor data comprising at least one of: a temperature of the tissue, an oxygen saturation of the tissue, a distance between light guides in proximity of the tissue, wherein the at least one processor obtains the reference fluorescence data from the digital library of fluorescence data, based also on the sensor data.

Optionally, the at least one processor is communicably coupled to the at least one sensor via a communication network. Examples of such a communication network are mentioned above. Examples of the temperature sensor include, but are not limited to, a thermocouple and a fiber-optic sensor (FOS). The FOS may be a fiber Bragg grating (FBG), a fluoroptic sensor, or similar. Examples of the oxygen saturation sensor include, but are not limited to, a tissue oxygen saturation (StO2) sensor, and a peripheral oxygen saturation (SpO2) sensor. Examples of the distance sensor can be an ultrasonic sensor, a fiber-optic sensor, and similar.

### DETAILED DESCRIPTION OF THE DRAWINGS

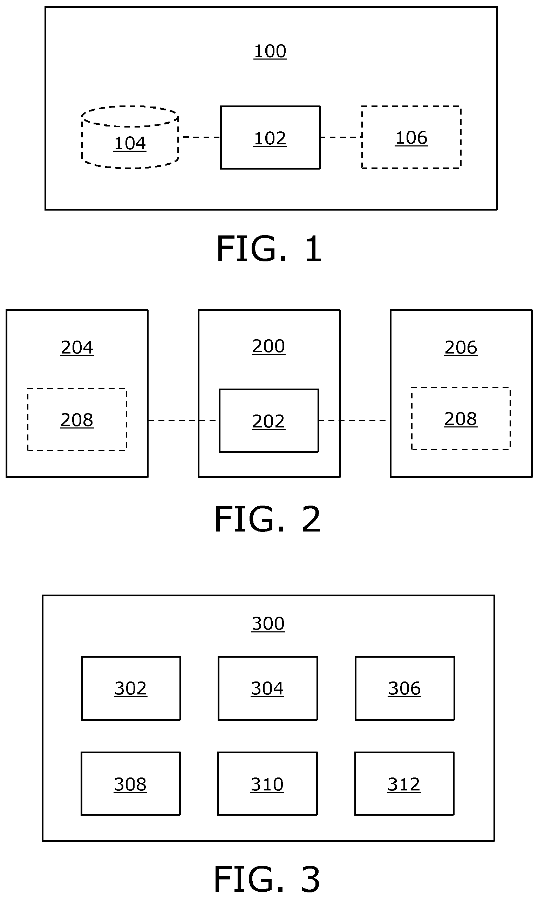

Referring to FIG. 1, illustrated is a block diagram of an architecture of a system **100** for monitoring a medical treatment of a tissue, in accordance with an embodiment of the present disclosure. The system **100** comprises at least one processor (depicted as a processor **102).** Optionally, the system **100** further comprises a data repository **104** communicably coupled to the processor **102.** Optionally, the system **100** further comprises at least one sensor (depicted as a sensor **106)** communicably coupled to the processor **102.**

Referring to FIG. 2, illustrated is an exemplary environment in which a system **200** for monitoring a medical treatment of a tissue is used, in accordance with an embodiment of the present disclosure. The system **200** comprises at least one processor (depicted as a processor **202).** The processor **202** is communicably coupled to a biomedical laser device **204** and/or a device **206** associated with an operator (not shown) of the biomedical laser device **204.** In this regard, the biomedical laser device **204** and/or the device **206** comprise at least one indicator (depicted as an indicator **208).**

Referring to FIG. 3, illustrated is an architecture of a biomedical laser device **300,** in accordance with an embodiment of the present disclosure. The biomedical laser device **300** comprises a laser light emitter **302,** at least one light guide (depicted, for example as two light guides **304** and **306),** a coupling arrangement **308** for optically coupling the laser light emitter **302** with the at least one light guide **304** and **306.** Optionally, the biomedical laser device **300** further comprises at least one indicator (depicted as an indicator **310).** Optionally, the biomedical laser device **300** further comprises at least one processor **312** of a system (not shown) for monitoring a medical treatment of a tissue.

FIGs. 1, 2, and 3 are merely examples, which should not unduly limit the scope of the claims herein. A person skilled in the art will recognize many variations, alternatives, and modifications of embodiments of the present disclosure. For example, the biomedical laser device **300** may comprise a single light guide.

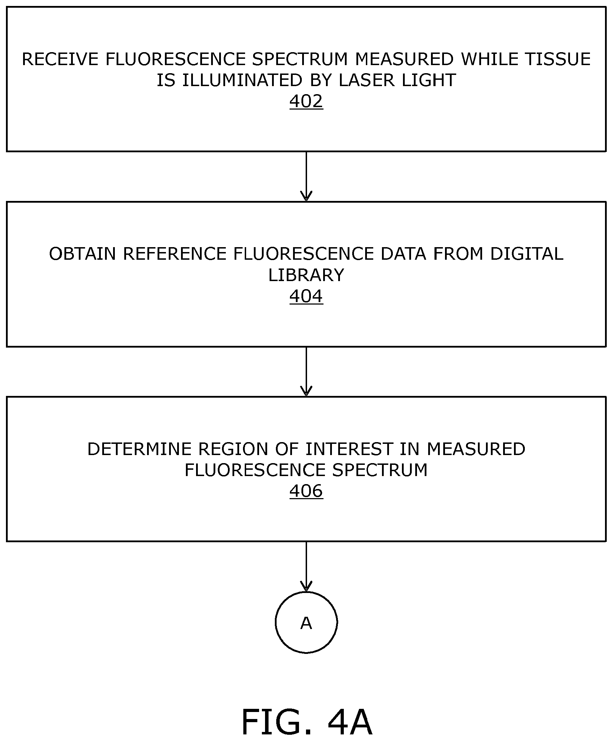

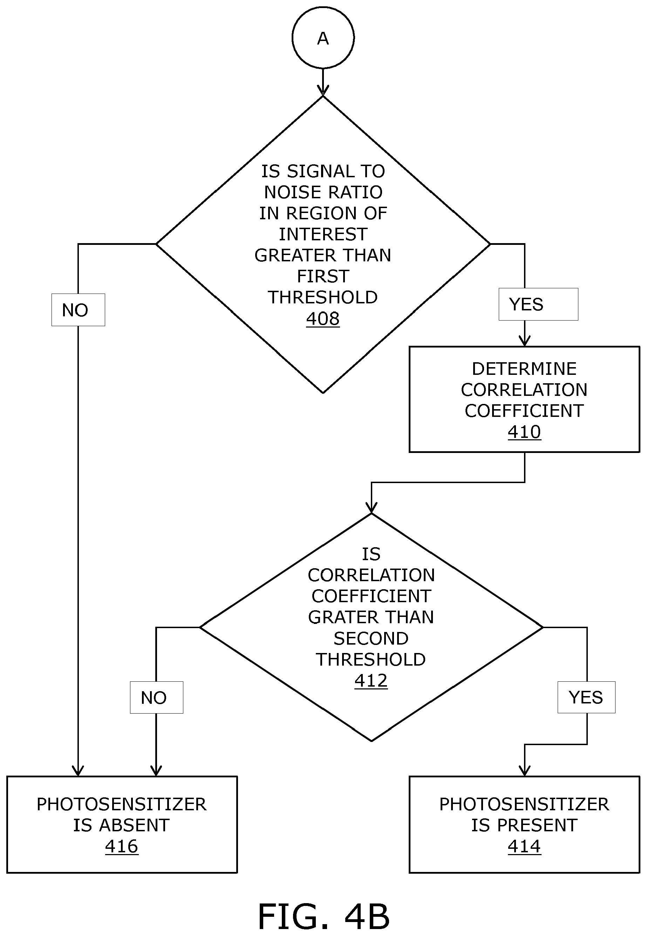

FIGs. 4A and 4B collectively illustrate a method for monitoring a medical treatment of a tissue, in accordance with an embodiment of the present disclosure. At step **402,** a fluorescence spectrum that is measured while the tissue is illuminated by laser light emitted by a biomedical laser device, is received. At step **404,** reference fluorescence data is obtained from a digital library of fluorescence data, based at least on a type of a photosensitizer that is required to be present in the tissue for the medical treatment. At step **406,** a region of interest in the measured fluorescence spectrum is determined, based on a reference fluorescence spectrum in the reference fluorescence data. At step **408,** it is determined whether a signal to noise ratio of the measured fluorescence spectrum in the region of interest is greater than a first threshold. When it is determined that the signal to noise ratio of the measured fluorescence spectrum in the region of interest is greater than the first threshold, at step **410,** there is determined a correlation coefficient that is indicative of a correlation between the region of interest in the measured fluorescence spectrum and the reference fluorescence spectrum. At step **412,** it is determined whether the correlation coefficient is greater than a second threshold. When it is determined that the correlation coefficient is greater than the second threshold, at step **414,** it is determined that the photosensitizer is present in the tissue. Notably, when it is determined that the signal to noise ratio of the measured fluorescence spectrum in the region of interest is not greater than the first threshold or when it is determined that the correlation coefficient is not greater than the second threshold, at step **416,** it is determined that the photosensitizer is absent in the tissue.

The aforementioned steps are only illustrative and other alternatives can also be provided where one or more steps are added, one or more steps are removed, or one or more steps are provided in a different sequence without departing from the scope of the claims herein. For example, the fluorescence spectrum received at the step **402** may be pre-processed by at least one of: removing dark spectrum from the measured fluorescence spectrum, removing ambient light spectrum from the measured fluorescence spectrum, low pass filtering of the measured fluorescence spectrum. In this regard, an output of the pre-processing step may be employed for calculation of the first threshold at a step prior to the step **408.**

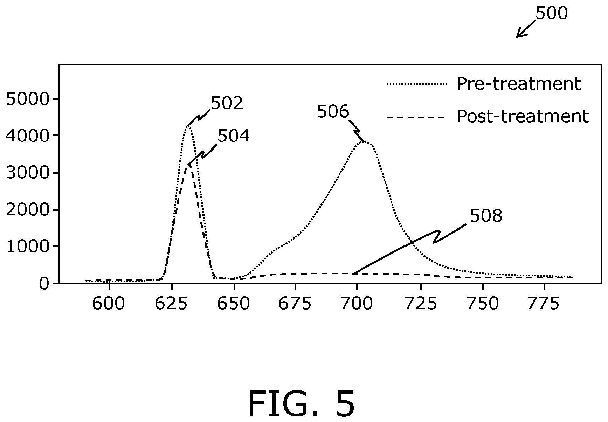

Referring to FIG. 5, illustrated are exemplary spectrums **500** corresponding to a pre-treatment phase (depicted as a dotted line) of a tissue and to a post-treatment phase (depicted as a dashed line) of the tissue, in accordance with an embodiment of the present disclosure. Peaks **502** and **504** correspond to laser light emitted by a biomedical laser device in the pre-treatment phase and in the post-treatment phase, respectively. Notably, both the peaks **502** and **504** are high since an intensity of the laser light emitted by the biomedical device in the pre-treatment phase and in the post-treatment phase, is high. Peaks **506** and **508** correspond to fluorescence emitted by a photosensitizer, in the pre-treatment phase and in the post-treatment phase, respectively. Notably, the peak **506** is much higher than the peak **508** since, in the pre-treatment phase, the photosensitizer is present in the tissue and emits strong fluorescence, but during treatment, the photosensitizer gets consumed and thus in the post-treatment phase, the photosensitizer is either minimally present or not present.

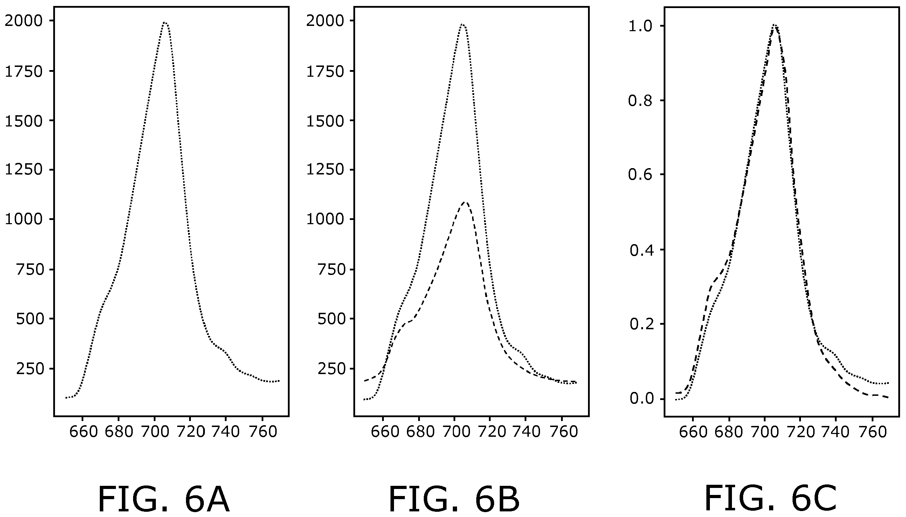

Referring to FIGs. 6A, 6B, and 6C, illustrated is a first exemplary set of graphs for determining whether a photosensitizer is present in a tissue, in accordance with an embodiment of the present disclosure. In FIGs. 6A-6C, the horizontal axis of the graphs represents wavelength whereas the vertical axis of the graphs represents intensity. FIG. 6A represents a region of interest (depicted as a dotted line) in a measured fluorescence spectrum, FIG. 6B represents a reference fluorescence spectrum (depicted as a dashed line) which is obtained from a digital library, as well as the region of interest in the measured fluorescence spectrum, and FIG. 6C represents the reference fluorescence spectrum and a normalized form of the region of interest in the measured fluorescence spectrum. Herein, let us consider that a signal to noise ratio of the measured fluorescence spectrum in the region of interest is greater than a first threshold. Then, a correlation between the region of interest in the measured fluorescence spectrum and the reference fluorescence spectrum is to be determined. As observed in FIG. 6C, the reference fluorescence spectrum and the normalized form of the region of interest in the measured fluorescence spectrum have a high level of similarity (in terms of peak shape, peak size, and similar) meaning that they are highly correlated with each other and hence a value of a correlation coefficient indicative of said correlation is also high. For example, in this case, the value of the correlation coefficient may be 0.995 (a maximum value of the correlation coefficient being 1, for example). Hence, when this correlation coefficient is greater than a second threshold, it is determined that a peak represented in FIG. 6A is really a fluorescence peak and that the photosensitizer is present in the tissue.

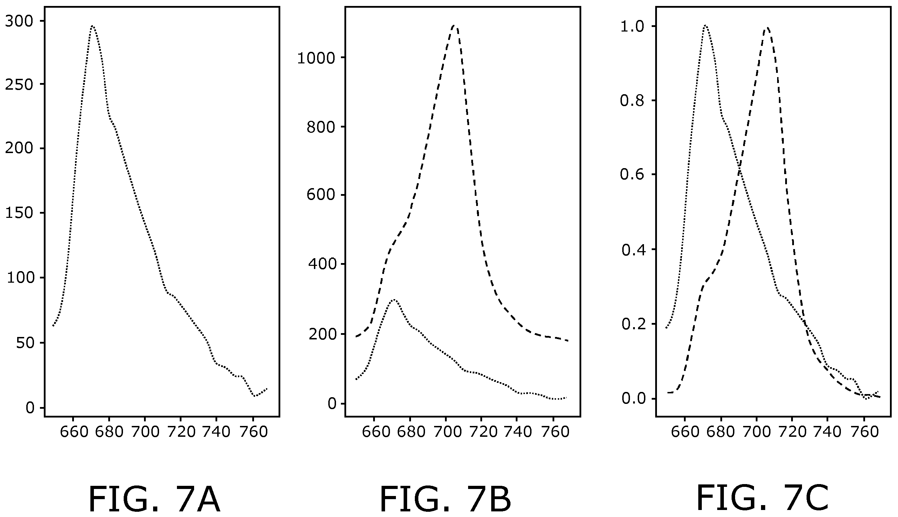

Referring to FIGs. 7A, 7B, and 7C, illustrated is a second exemplary set of graphs for determining whether a photosensitizer is present in a tissue, in accordance with an embodiment of the present disclosure. In FIGs. 7A-7C, the horizontal axis of the graphs represents wavelength whereas the vertical axis of the graphs represents intensity. FIG. 7A represents a region of interest (depicted as a dotted line) in a measured fluorescence spectrum, FIG. 7B represents a reference fluorescence spectrum (depicted as a dashed line) which is obtained from a digital library, as well as the region of interest in the measured fluorescence spectrum, and FIG. 7C represents the reference fluorescence spectrum and a normalized form of the region of interest in the measured fluorescence spectrum. Herein, let us consider that a signal to noise ratio of the measured fluorescence spectrum in the region of interest is greater than a first threshold. Then, a correlation between the region of interest in the measured fluorescence spectrum and the reference fluorescence spectrum is to be determined. As observed in FIG. 7C, the reference fluorescence spectrum and the normalized form of the region of interest in the measured fluorescence spectrum have a low level of similarity (in terms of peak shape, peak size, and similar) meaning that they are quite uncorrelated with each other and hence a value of a correlation coefficient indicative of said correlation is also low. For example, in this case, the value of correlation may be 0.404 (a maximum value of correlation coefficient being 1, for example). Hence, when this correlation coefficient is lesser than or equal to a second threshold, it is determined that the photosensitizer is absent in the tissue.

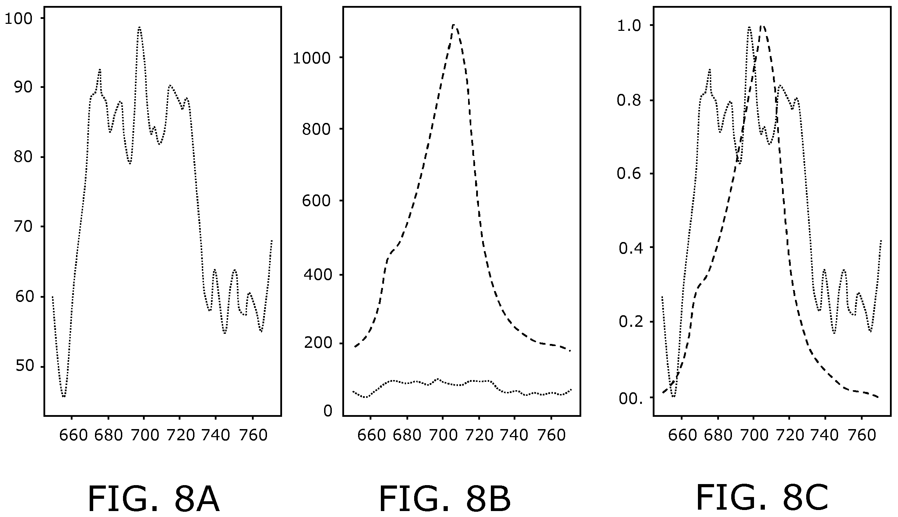

Referring to FIGs. 8A, 8B, and 8C, illustrated is a third exemplary set of graphs for determining whether a photosensitizer is present in a tissue, in accordance with an embodiment of the present disclosure. In FIGs. 8A-8C, the horizontal axis of the graphs represents wavelength whereas the vertical axis of the graphs represents intensity. FIG. 8A represents a region of interest (depicted as a dotted line) in a measured fluorescence spectrum, FIG. 8B represents a reference fluorescence spectrum (depicted as a dashed line) which is obtained from a digital library, as well as the region of interest in the measured fluorescence spectrum, and FIG. 8C represents the reference fluorescence spectrum and a normalized form of the region of interest in the measured fluorescence spectrum. Herein, let us consider that a signal to noise ratio of the measured fluorescence spectrum in the region of interest is greater than a first threshold. Then, a correlation between the region of interest in the measured fluorescence spectrum and the reference fluorescence spectrum is to be determined. As observed in FIG. 8C, the reference fluorescence spectrum and the normalized form of the region of interest in the measured fluorescence spectrum have a low level of similarity (in terms of peak shape, peak size, and similar) meaning that they are less correlated with each other and hence a value of a correlation coefficient indicative of said correlation is also low. Moreover, in FIGs. 8A and 8C, the region of interest in the measured fluorescence spectrum includes quite a lot of noise. For example, in this case, the value of correlation may be 0.783 (a maximum value of correlation coefficient being 1, for example). Hence, when this correlation coefficient is lesser than or equal to a second threshold, it is determined that the photosensitizer is absent in the tissue.

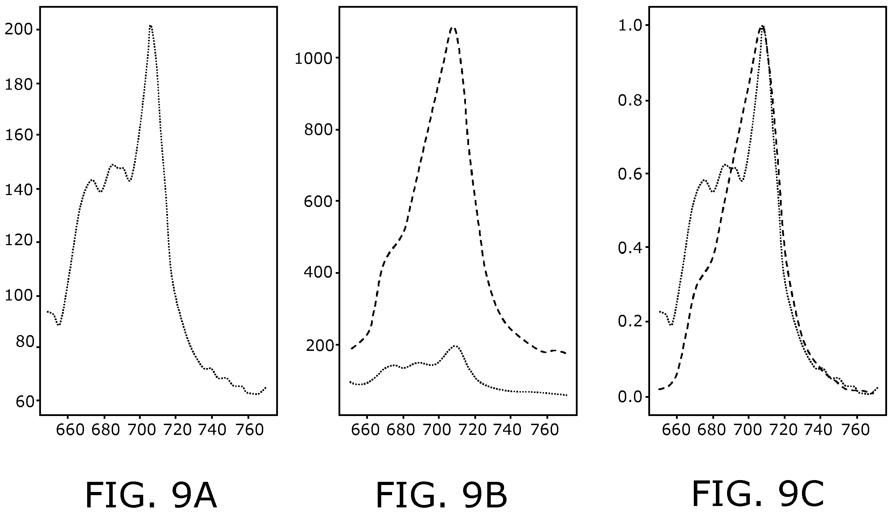

Referring to FIGs. 9A, 9B, and 9C, illustrated is a fourth exemplary set of graphs for determining whether a photosensitizer is present in a tissue, in accordance with an embodiment of the present disclosure. In FIGs. 9A-9C, the horizontal axis of the graphs represents wavelength whereas the vertical axis of the graphs represents intensity. FIG. 9A represents a region of interest (depicted as a dotted line) in a measured fluorescence spectrum, FIG. 9B represents a reference fluorescence spectrum (depicted as a dashed line) which is obtained from a digital library, as well as the region of interest in the measured fluorescence spectrum, and FIG. 9C represents the reference fluorescence spectrum and a normalized form of the region of interest in the measured fluorescence spectrum. Herein, let us consider that a signal to noise ratio of the measured fluorescence spectrum in the region of interest is greater than a first threshold. Then, a correlation between the region of interest in the measured fluorescence spectrum and the reference fluorescence spectrum is to be determined. As observed in FIG. 9C, the reference fluorescence spectrum and the normalized form of the region of interest in the measured fluorescence spectrum have a high level of similarity (in terms of peak shape, peak size, and similar) meaning that they are highly correlated with each other and hence a value of a correlation coefficient indicative of said correlation is also high. For example, in this case, the value of the correlation coefficient may be 0.92 (a maximum value of the correlation coefficient being 1, for example). Hence, when this correlation coefficient is greater than a second threshold, it is determined that a peak represented in FIG. 9A is really a fluorescence peak and that the photosensitizer is present in the tissue.

FIGs. 6A-6C, 7A-7C, 8A-8C and 9A-9C are merely examples, which should not unduly limit the scope of the claims herein. A person skilled in the art will recognize many variations, alternatives, and modifications of embodiments of the present disclosure. For example, in FIGs. 6C, 7C, 8C and 9C, there may be represented the region of interest in the measured fluorescence spectrum and a normalized form of the reference fluorescence spectrum.

Modifications to embodiments of the present disclosure described in the foregoing are possible without departing from the scope of the present disclosure as defined by the accompanying claims. Expressions such as "including", "comprising", "incorporating", "have", "is" used to describe the present disclosure are intended to be construed in a non-exclusive manner, namely allowing for items, components or elements not explicitly described also to be present. Reference to the singular is also to be construed to relate to the plural.

## Claims

1. A method for monitoring a medical treatment of a tissue, the method comprising:
- receiving, by at least one processor (102, 202, 312), a fluorescence spectrum that is measured while the tissue is illuminated by laser light emitted by a biomedical laser device (204, 300), wherein the fluorescence spectrum corresponds to a variation of an intensity of light emitted by the photosensitizer when the tissue is illuminated by laser light emitted by the biomedical laser device (204, 300);
- obtaining, by the at least one processor (102, 202, 312), reference fluorescence data from a digital library of fluorescence data, based at least on a type of a photosensitizer that is required to be present in the tissue for the medical treatment;
- determining, by the at least one processor (102, 202, 312), a region of interest in the measured fluorescence spectrum, based on a reference fluorescence spectrum in the reference fluorescence data;
- determining, by the at least one processor (102, 202, 312), whether a signal to noise ratio of the measured fluorescence spectrum in the region of interest is greater than a first threshold;
- when it is determined that the signal to noise ratio of the measured fluorescence spectrum in the region of interest is greater than the first threshold, determining, by the at least one processor (102, 202, 312), a correlation coefficient that is indicative of a correlation between the region of interest in the measured fluorescence spectrum and the reference fluorescence spectrum;
- determining, by the at least one processor (102, 202, 312), whether the correlation coefficient is greater than a second threshold; and
- when it is determined that the correlation coefficient is greater than the second threshold, determining, by the at least one processor (102, 202, 312), that the photosensitizer is present in the tissue.

2. A method according to claim 1, wherein when it is determined that the photosensitizer is present in the tissue, the method further comprises providing an indication of the photosensitizer being present in the tissue, the indication being at least one of: a visual indication, an audio indication, a haptic indication.

3. A method according to claim 1 or 2, wherein when it is determined that the photosensitizer is present in the tissue, the method further comprises sending measured fluorescence data to a data repository (104) at which the digital library of fluorescence data is maintained, for storing the measured fluorescence data in the digital library, wherein the measured fluorescence data comprises the measured fluorescence spectrum, the type of the photosensitizer present in the tissue, and the correlation coefficient that is determined.

4. A method according to any of the preceding claims, wherein when it is determined that the signal to noise ratio of the measured fluorescence spectrum in the region of interest is lesser than or equal to the first threshold, the method further comprises determining that the photosensitizer is absent in the tissue.

5. A method according to any of the preceding claims, wherein when it is determined that the correlation coefficient is lesser than or equal to the second threshold, the method further comprises determining that the photosensitizer is absent in the tissue.

6. A method according to any of the preceding claims, further comprising processing the measured fluorescence spectrum, by at least one of:
- removing dark spectrum from the measured fluorescence spectrum,
- removing ambient light spectrum from the measured fluorescence spectrum,
- low pass filtering of the measured fluorescence spectrum.

7. A method according to any of the preceding claims, further comprising receiving sensor data that is measured by at least one sensor (106) arranged in proximity of the tissue, the sensor data comprising at least one of: a temperature of the tissue, an oxygen saturation of the tissue, a distance between light guides (304, 306) in proximity of the tissue,
wherein the step of obtaining the reference fluorescence data from the digital library of fluorescence data is based also on the sensor data.

8. A method according to any of the preceding claims, further comprising creating the digital library of fluorescence data using at least historical measured fluorescence spectrums, wherein the historical measured fluorescence spectrums are measured historically by administering different types of photosensitisers in the tissue.

9. A method according to claim 8, wherein the historical measured fluorescence spectrums are measured historically for at least one of: different temperatures of the tissue, different oxygen saturations in the tissue, different distances between light guides (304, 306) in proximity of the tissue.

10. A system (100, 200) for monitoring a medical treatment of a tissue, the system comprising at least one processor (102, 202, 312) configured to:
- receive a fluorescence spectrum that is measured while the tissue is illuminated by laser light emitted by a biomedical laser device (204, 300), wherein the fluorescence spectrum corresponds to a variation of an intensity of light emitted by the photosensitizer when the tissue is illuminated by laser light emitted by the biomedical laser device (204, 300);
- obtain reference fluorescence data from a digital library of fluorescence data, based at least on a type of a photosensitizer that is required to be present in the tissue for the medical treatment;
- determine a region of interest in the measured fluorescence spectrum, based on a reference fluorescence spectrum in the reference fluorescence data;
- determine whether a signal to noise ratio of the measured fluorescence spectrum in the region of interest is greater than a first threshold;
- when it is determined that the signal to noise ratio of the measured fluorescence spectrum in the region of interest is greater than the first threshold, determine a correlation coefficient that is indicative of a correlation between the region of interest in the measured fluorescence spectrum and the reference fluorescence spectrum;
- determine whether the correlation coefficient is greater than a second threshold; and
- when it is determined that the correlation coefficient is greater than the second threshold, determine that the photosensitizer is present in the tissue.

11. A system (100, 200) according to claim 10, further comprising a data repository (104) communicably coupled to the at least one processor (102, 202, 312), wherein the digital library of fluorescence data is maintained at the data repository.

12. A system (100, 200) according to claim 10 or 11, wherein the at least one processor (102, 202, 312) is communicably coupled to the biomedical laser device (204, 300) and/or a device (206) associated with an operator of the biomedical laser device, and wherein when it is determined that the photosensitizer is present in the tissue, the at least one processor is further configured to provide an indication of the photosensitizer being present in the tissue on the biomedical laser device and/or the device associated with an operator of the biomedical laser device, the indication being at least one of: a visual indication, an audio indication, a haptic indication.

13. A system (100, 200) according to any of claims 10-12, wherein the biomedical laser device (204, 300) comprises the at least one processor (102, 202, 312).

14. A system (100, 200) according to any of claims 10-13, further comprising at least one sensor (106) communicably coupled to the at least one processor (102, 202, 312), the at least one sensor comprising at least one of: a temperature sensor, an oxygen saturation sensor, a distance sensor, the at least one sensor being arranged in proximity of the tissue, and wherein the at least one processor is further configured to receive sensor data that is measured by the at least one sensor, the sensor data comprising at least one of: a temperature of the tissue, an oxygen saturation of the tissue, a distance between light guides (304, 306) in proximity of the tissue,
wherein the at least one processor obtains the reference fluorescence data from the digital library of fluorescence data, based also on the sensor data.

## Patentansprüche

1. Verfahren zum Überwachen einer medizinischen Behandlung von Gewebe, wobei das Verfahren umfasst:
- Empfangen, durch mindestens einen Prozessor (102, 202, 312), eines Fluoreszenzspektrums, das gemessen wird, während das Gewebe durch Laserlicht beleuchtet wird, das durch eine biomedizinische Laservorrichtung (204, 300) emittiert wird, wobei das Fluoreszenzspektrum einer Variation einer Intensität von Licht entspricht, das durch den Photosensibilisator emittiert wird, wenn das Gewebe mit Laserlicht beleuchtet wird, das durch die biomedizinische Laservorrichtung (204, 300) emittiert wird;
- Erhalten, durch den mindestens einen Prozessor (102, 202, 312), von Referenzfluoreszenzdaten aus einer digitalen Bibliothek von Fluoreszenzdaten basierend mindestens auf einer Art eines Photosensibilisators, der für die medizinische Behandlung in dem Gewebe vorhanden sein muss;
- Bestimmen, durch den mindestens einen Prozessor (102, 202, 312), eines interessierenden Bereichs in dem gemessenen Fluoreszenzspektrum basierend auf einem Referenzfluoreszenzspektrum in den Referenzfluoreszenzdaten;
- Bestimmen, durch den mindestens einen Prozessor (102, 202, 312), ob ein Signal-Rausch-Verhältnis des gemessenen Fluoreszenzspektrums in dem interessierenden Bereich größer als ein erster Schwellenwert ist;
- wenn festgestellt wird, dass das Signal-Rausch-Verhältnis des gemessenen Fluoreszenzspektrums in dem interessierenden Bereich größer als der erste Schwellenwert ist, Bestimmen, durch den mindestens einen Prozessor (102, 202, 312), eines Korrelationskoeffizienten, der bezeichnend für eine Korrelation zwischen dem interessierenden Bereich in dem gemessenen Fluoreszenzspektrum und dem Referenzfluoreszenzspektrum ist;
- Bestimmen, durch den mindestens einen Prozessor (102, 202, 312), ob der Korrelationskoeffizient größer als ein zweiter Schwellenwert ist; und
- wenn festgestellt wird, dass der Korrelationskoeffizient größer als der zweite Schwellenwert ist, Feststellen, durch den mindestens einen Prozessor (102, 202, 312), dass der Photosensibilisator in dem Gewebe vorhanden ist.

2. Verfahren nach Anspruch 1, wobei, wenn festgestellt wird, dass der Photosensibilisator in dem Gewebe vorhanden ist, das Verfahren ferner das Bereitstellen einer Anzeige umfasst, dass der Photosensibilisator in dem Gewebe vorhanden ist, wobei die Anzeige mindestens eine ist von: einer visuellen Anzeige, einer akustischen Anzeige, einer haptischen Anzeige.

3. Verfahren nach Anspruch 1 oder 2, wobei, wenn festgestellt wird, dass der Photosensibilisator in dem Gewebe vorhanden ist, das Verfahren ferner das Senden gemessener Fluoreszenzdaten an einen Datenspeicher (104), in dem die digitale Bibliothek von Fluoreszenzdaten verwaltet wird, zum Speichern der gemessenen Fluoreszenzdaten in der digitalen Bibliothek umfasst, wobei die gemessenen Fluoreszenzdaten das gemessene Fluoreszenzspektrum, die Art des in dem Gewebe vorhandenen Photosensibilisators und den Korrelationskoeffizienten umfasst, der bestimmt wird.

4. Verfahren nach einem der vorstehenden Ansprüche, wobei, wenn festgestellt wird, dass das Signal-Rausch-Verhältnis des gemessenen Fluoreszenzspektrums in dem interessierenden Bereich kleiner oder gleich dem ersten Schwellenwert ist, das Verfahren ferner das Feststellen umfasst, dass der Photosensibilisator nicht in dem Gewebe vorhanden ist.

5. Verfahren nach einem der vorstehenden Ansprüche, wobei, wenn festgestellt wird, dass der Korrelationskoeffizient kleiner oder gleich dem zweiten Schwellenwert ist, das Verfahren ferner das Feststellen umfasst, dass der Photosensibilisator nicht in dem Gewebe vorhanden ist.

6. Verfahren nach einem der vorstehenden Ansprüche, ferner umfassend das Verarbeiten des gemessenen Fluoreszenzspektrums durch mindestens eines von:
- Entfernen des Dunkelspektrums aus dem gemessenen Fluoreszenzspektrum,
- Entfernen des Umgebungslichtspektrums aus dem gemessenen Fluoreszenzspektrum,
- Tiefpassfilterung des gemessenen Fluoreszenzspektrums.

7. Verfahren nach einem der vorstehenden Ansprüche, ferner umfassend das Empfangen von Sensordaten, die durch mindestens einen Sensor (106) gemessen werden, der in der Nähe des Gewebes angeordnet ist, wobei die Sensordaten mindestens eines umfassen von: einer Temperatur des Gewebes, einer Sauerstoffsättigung des Gewebes, einem Abstand zwischen Lichtleitern (304, 306) in der Nähe des Gewebes,
wobei der Schritt des Erhaltens der Referenzfluoreszenzdaten aus der digitalen Bibliothek von Fluoreszenzdaten auch auf den Sensordaten basiert.

8. Verfahren nach einem der vorstehenden Ansprüche, ferner umfassend das Erstellen der digitalen Bibliothek von Fluoreszenzdaten unter Verwendung von mindestens historischen gemessenen Fluoreszenzspektren, wobei die historischen gemessenen Fluoreszenzspektren historisch durch Verabreichen verschiedener Arten von Photosensibilisatoren in das Gewebe gemessen werden.

9. Verfahren nach Anspruch 8, wobei die historischen gemessenen Fluoreszenzspektren historisch gemessen werden für mindestens eines von: unterschiedlichen Temperaturen des Gewebes, unterschiedlichen Sauerstoffsättigungen in dem Gewebe, unterschiedlichen Abständen zwischen Lichtleitern (304, 306) in der Nähe des Gewebes.

10. System (100, 200) zum Überwachen einer medizinischen Behandlung eines Gewebes, wobei das System mindestens einen Prozessor (102, 202, 312) umfasst, der konfiguriert ist zum:
- Empfangen eines Fluoreszenzspektrums, das gemessen wird, während das Gewebe durch Laserlicht beleuchtet wird, das durch eine biomedizinische Laservorrichtung (204, 300) emittiert wird, wobei das Fluoreszenzspektrum einer Variation einer Intensität von Licht entspricht, das durch den Photosensibilisator emittiert wird, wenn das Gewebe durch Laserlicht beleuchtet wird, das durch die biomedizinische Laservorrichtung (204, 300) emittiert wird;
- Erhalten von Referenzfluoreszenzdaten aus einer digitalen Bibliothek von Fluoreszenzdaten mindestens basierend auf einer Art eines Photosensibilisators, der für die medizinische Behandlung in dem Gewebe vorhanden sein muss;
- Bestimmen eines interessierenden Bereichs in dem gemessenen Fluoreszenzspektrum basierend auf einem Referenzfluoreszenzspektrum in den Referenzfluoreszenzdaten;
- Bestimmen, ob ein Signal-Rausch-Verhältnis des gemessenen Fluoreszenzspektrums in dem interessierenden Bereich größer als ein erster Schwellenwert ist;
- wenn festgestellt wird, dass das Signal-Rausch-Verhältnis des gemessenen Fluoreszenzspektrums in dem interessierenden Bereich größer als der erste Schwellenwert ist, Bestimmen eines Korrelationskoeffizienten, der bezeichnend für eine Korrelation zwischen dem interessierenden Bereich in dem gemessenen Fluoreszenzspektrum und dem Referenzfluoreszenzspektrum ist;
- Bestimmen, ob der Korrelationskoeffizient größer als ein zweiter Schwellenwert ist; und
- wenn festgestellt wird, dass der Korrelationskoeffizient größer als der zweite Schwellenwert ist, Feststellen, dass der Photosensibilisator in dem Gewebe vorhanden ist.

11. System (100, 200) nach Anspruch 10, ferner umfassend einen Datenspeicher (104), der zu Kommunikationszwecken mit dem mindestens einen Prozessor (102, 202, 312) gekoppelt ist, wobei die digitale Bibliothek von Fluoreszenzdaten in dem Datenspeicher verwaltet wird.

12. System (100, 200) nach Anspruch 10 oder 11, wobei der mindestens eine Prozessor (102, 202, 312) zu Kommunikationszwecken mit der biomedizinischen Laservorrichtung (204, 300) und/oder einer Vorrichtung (206) gekoppelt ist, die einem Bediener der biomedizinischen Laservorrichtung zugeordnet ist, und wobei, wenn festgestellt wird, dass der Photosensibilisator in dem Gewebe vorhanden ist, der mindestens eine Prozessor ferner konfiguriert ist, um eine Anzeige des Vorhandenseins des Photosensibilisators in dem Gewebe auf der biomedizinischen Laservorrichtung und/oder der Vorrichtung bereitzustellen, die einem Bediener der biomedizinischen Laservorrichtung zugeordnet ist, wobei die Anzeige mindestens eine ist von: einer visuellen Anzeige, einer akustischen Anzeige, einer haptischen Anzeige.

13. System (100, 200) nach einem der Ansprüche 10 bis 12, wobei die biomedizinische Laservorrichtung (204, 300) den mindestens einen Prozessor (102, 202, 312) umfasst.

14. System (100, 200) nach einem der Ansprüche 10 bis 13, ferner umfassend mindestens einen Sensor (106), der zu Kommunikationszwecken mit dem mindestens einen Prozessor (102, 202, 312) gekoppelt ist, wobei der mindestens eine Sensor mindestens eines umfasst von: einem Temperaturfühler, einem Sauerstoffsättigungssensor, einem Abstandssensor, wobei der mindestens eine Sensor in der Nähe des Gewebes angeordnet ist und wobei der mindestens eine Prozessor ferner konfiguriert ist, um Sensordaten zu empfangen, die durch den mindestens einen Sensor gemessen werden, wobei die Sensordaten mindestens eines umfassen von: einer Temperatur des Gewebes, einer Sauerstoffsättigung des Gewebes, einem Abstand zwischen Lichtleitern (304, 306) in der Nähe des Gewebes,
wobei der mindestens eine Prozessor die Referenzfluoreszenzdaten aus der digitalen Bibliothek von Fluoreszenzdaten auch basierend auf den Sensordaten erhält.

## Revendications

1. Procédé permettant de surveiller un traitement médical d'un tissu, le procédé comprenant :
- la réception, par au moins un processeur (102, 202, 312), d'un spectre de fluorescence qui est mesuré pendant que le tissu est éclairé par une lumière laser émise par un dispositif laser biomédical (204, 300), dans lequel le spectre de fluorescence correspond à une variation d'une intensité de lumière émise par le photosensibilisateur lorsque le tissu est éclairé par une lumière laser émise par le dispositif laser biomédical (204, 300) ;
- l'obtention, par l'au moins un processeur (102, 202, 312), de données de fluorescence de référence auprès d'une bibliothèque numérique de données de fluorescence, sur la base au moins d'un type de photosensibilisateur qui doit être présent dans le tissu pour le traitement médical ;
- la détermination, par l'au moins un processeur (102, 202, 312), d'une région d'intérêt dans le spectre de fluorescence mesuré, sur la base d'un spectre de fluorescence de référence dans les données de fluorescence de référence ;
- le fait de déterminer, par l'au moins un processeur (102, 202, 312), si un rapport signal sur bruit du spectre de fluorescence mesuré dans la région d'intérêt est supérieur à un premier seuil ;
- lorsqu'il est déterminé que le rapport signal sur bruit du spectre de fluorescence mesuré dans la région d'intérêt est supérieur au premier seuil, la détermination, par l'au moins un processeur (102, 202, 312), un coefficient de corrélation qui indique une corrélation entre la région d'intérêt dans le spectre de fluorescence mesuré et le spectre de fluorescence de référence ;
- le fait de déterminer, par l'au moins un processeur (102, 202, 312), si le coefficient de corrélation est supérieur à un second seuil ; et
- lorsqu'il est déterminé que le coefficient de corrélation est supérieur au second seuil, le fait de déterminer, par l'au moins un processeur (102, 202, 312), que le photosensibilisateur est présent dans le tissu.

2. Procédé selon la revendication 1, dans lequel, lorsqu'il est déterminé que le photosensibilisateur est présent dans le tissu, le procédé comprend en outre la fourniture d'une indication de la présence du photosensibilisateur dans le tissu, l'indication étant au moins l'une parmi : une indication visuelle, une indication audio, une indication haptique.

3. Procédé selon la revendication 1 ou 2, dans lequel, lorsqu'il est déterminé que le photosensibilisateur est présent dans le tissu, le procédé comprend en outre l'envoi de données de fluorescence mesurées à un référentiel de données (104) au niveau duquel la bibliothèque numérique de données de fluorescence est conservée, pour stocker les données de fluorescence mesurées dans la bibliothèque numérique, dans lequel les données de fluorescence mesurées comprennent le spectre de fluorescence mesuré, le type du photosensibilisateur présent dans le tissu, et le coefficient de corrélation qui est déterminé.

4. Procédé selon l'une quelconque des revendications précédentes, dans lequel, lorsqu'il est déterminé que le rapport signal sur bruit du spectre de fluorescence mesuré dans la région d'intérêt est inférieur ou égal au premier seuil, le procédé comprend en outre le fait de déterminer que le photosensibilisateur est absent dans le tissu.

5. Procédé selon l'une quelconque des revendications précédentes, dans lequel, lorsqu'il est déterminé que le coefficient de corrélation est inférieur ou égal au second seuil, le procédé comprend en outre le fait de déterminer que le photosensibilisateur est absent dans le tissu.

6. Procédé selon l'une quelconque des revendications précédentes, comprenant en outre le traitement du spectre de fluorescence mesuré, par au moins l'un parmi :
- le retrait d'un spectre d'obscurité du spectre de fluorescence mesuré,
- le retrait du spectre de lumière ambiante du spectre de fluorescence mesuré,
- le filtrage passe-bas du spectre de fluorescence mesuré.

7. Procédé selon l'une quelconque des revendications précédentes, comprenant en outre la réception de données de capteur qui sont mesurées par au moins un capteur (106) agencé à proximité du tissu, les données de capteur comprenant au moins l'une parmi : une température du tissu, une saturation en oxygène du tissu, une distance entre des guides de lumière (304, 306) à proximité du tissu,
dans lequel l'étape consistant à obtenir des données de fluorescence de référence auprès de la bibliothèque numérique de données de fluorescence est également basée sur les données de capteur.

8. Procédé selon l'une quelconque des revendications précédentes, comprenant en outre la création de la bibliothèque numérique de données de fluorescence à l'aide d'au moins des spectres de fluorescence mesurés historiques, dans lequel les spectres de fluorescence mesurés historiques sont mesurés historiquement en administrant différents types de photosensibilisateurs dans le tissu.

9. Procédé selon la revendication 8, dans lequel les spectres de fluorescence mesurés historiques sont mesurés historiquement pour au moins l'une parmi : différentes températures du tissu, différentes saturations en oxygène dans le tissu, différentes distances entre des guides de lumière (304, 306) à proximité du tissu.

10. Système (100, 200) permettant de surveiller un traitement médical d'un tissu, le système comprenant au moins un processeur (102, 202, 312) configuré pour :
- recevoir un spectre de fluorescence qui est mesuré pendant que le tissu est éclairé par une lumière laser émise par un dispositif laser biomédical (204, 300), dans lequel le spectre de fluorescence correspond à une variation d'une intensité de lumière émise par le photosensibilisateur lorsque le tissu est éclairé par une lumière laser émise par le dispositif laser biomédical (204, 300) ;
- obtenir des données de fluorescence de référence auprès d'une bibliothèque numérique de données de fluorescence, sur la base au moins d'un type de photosensibilisateur qui doit être présent dans le tissu pour le traitement médical ;
- déterminer une région d'intérêt dans le spectre de fluorescence mesuré, sur la base d'un spectre de fluorescence de référence dans les données de fluorescence de référence ;
- déterminer si un rapport signal sur bruit du spectre de fluorescence mesuré dans la région d'intérêt est supérieur à un premier seuil ;
- lorsqu'il est déterminé que le rapport signal sur bruit du spectre de fluorescence mesuré dans la région d'intérêt est supérieur au premier seuil, déterminer un coefficient de corrélation qui indique une corrélation entre la région d'intérêt dans le spectre de fluorescence mesuré et le spectre de fluorescence de référence ;
- déterminer si le coefficient de corrélation est supérieur à un second seuil ; et
- lorsqu'il est déterminé que le coefficient de corrélation est supérieur au second seuil, déterminer que le photosensibilisateur est présent dans le tissu.

11. Système (100, 200) selon la revendication 10, comprenant en outre un référentiel de données (104) couplé en communication à l'au moins un processeur (102, 202, 312), dans lequel la bibliothèque numérique de données de fluorescence est conservée dans le référentiel de données.

12. Système (100, 200) selon la revendication 10 ou 11, dans lequel l'au moins un processeur (102, 202, 312) est couplé en communication au dispositif laser biomédical (204, 300) et/ou à un dispositif (206) associé à un opérateur du dispositif laser biomédical, et dans lequel, lorsqu'il est déterminé que le photosensibilisateur est présent dans le tissu, l'au moins un processeur est en outre configuré pour fournir une indication de la présence du photosensibilisateur dans le tissu sur le dispositif laser biomédical et/ou le dispositif associé à un opérateur du dispositif laser biomédical, l'indication étant au moins l'une parmi : une indication visuelle, une indication audio, une indication haptique.

13. Système (100, 200) selon l'une quelconque des revendications 10 à 12, dans lequel le dispositif laser biomédical (204, 300) comprend l'au moins un processeur (102, 202, 312).

14. Système (100, 200) selon l'une quelconque des revendications 10 à 13, comprenant en outre au moins un capteur (106) couplé en communication à l'au moins un processeur (102, 202, 312), l'au moins un capteur comprenant au moins l'un parmi : un capteur de température, un capteur de saturation en oxygène, un capteur de distance, l'au moins un capteur étant agencé à proximité du tissu, et dans lequel l'au moins un processeur est en outre configuré pour recevoir des données de capteur qui sont mesurées par l'au moins un capteur, les données de capteur comprenant au moins l'une parmi : une température du tissu, une saturation en oxygène du tissu, une distance entre des guides de lumière (304, 306) à proximité du tissu,
dans lequel l'au moins un processeur obtient les données de fluorescence de référence auprès de la bibliothèque numérique de données de fluorescence, sur la base également des données de capteur.
